# EUROPEAN PATENT APPLICATION

(11) **EP 4 001 785 A1**
(43) Date of publication of application: **25.05.2022**
(21) Application number: 20208188.1
(22) Date of filing: 17.11.2020
(51) Int. Cl.: F24F 3/16, F24F 8/10, F24F 8/167, F24F 8/22, A61L 9/20

(54) **INSERT DEVICE FOR AN AIR CONDITIONING INSTALLATION AND AIR CONDITIONING INSTALLATION WITH INSERT DEVICE**

(71) Applicant: Calistair SAS, 77250 Moret Loing et Orvanne (FR)
(72) Inventor: Taranto, Jérôme, 77130 Dormelles (FR)
(74) Representative: Gramm, Lins & Partner Patent- und Rechtsanwälte PartGmbB

(57) **Abstract**

The invention refers to a Insert device for an air conditioning installation which air conditioning installation comprises an air handling unit (1) guiding air flowing in a given flow direction where the air handling unit (1) has a casing with a predetermined cross section in which at least a filter (5, 7) is housed, wherein the insert device comprises a frame casing (8) with an outer closed periphery shaped to be mounted within a predetermined cross section of the casing of the air handling unit (1) and has end faces (9) open for the air flow, at each of the end faces (9) an air-permeable catalytic grid structure (12) is held comprising a carrier grid (17) and a coating with a catalytic material, that the catalytic material is a mixture comprising an absorbent, a first catalyst activatable by electromagnetic radiation, and a second catalyst being activated at low temperature, and that the frame casing (8) in addition holds an electromagnetic radiation source device between the catalytic grid structures (12) at its end faces (9).

## Description

The invention relates to an insert device for an air conditioning installation which air conditioning installation comprises an air handling unit guiding air flowing in a given flow direction, wherein the air handling unit has a casing with a predetermined cross section in which at least a filter is housed.

Furthermore, the invention relates to an air conditioning installation having an insert device.

Air conditioning installations are used in closed rooms where a number of humans or animals are present for living, working and/or meeting. In order to avoid an accumulation of dust the air conditioning installation serves for removing air from the room and for replacing it after filtering. In many cases at least some of the air removed from the room is replaced by fresh air drawn from the outside of the room for replacing accumulated CO₂ by O₂. If necessary, the fresh air may be heated or cooled.

Most of the air conditioning installations are suitable for removing particles from the air within the room so that at least a certain amount of the air within the room may be recirculated. However, most of that installations are designed to trap particles and chemical pollutants by further units which have to be replaced in time to avoid their ineffectiveness due to overload or fouling.

In this specification air conditioning installations are meant to include fixed installations where the air handling unit is connected to an air guiding tubing as well as installations where the air handling unit comprises a cabinet which may be positioned deliberately. Said cabinet may be used as a mobile air conditioning installation having input and output openings which may be in direct connection to the ambient air or, as an alternative, having at least one opening connected to a tube for sucking air to be cleaned from a room or for distributing cleaned air.

Air conditioning installations are known which are able to not only absorb but also destroy chemical and even microbiological pollutants. They use cabinets of a pretty large size as an air handling unit to which the air guiding tubing is connected. In times where there is an acute threat due to bacteria, viruses, and fungi the usual air conditioning installations can reasonably not be used if they only recirculate the air within a room. As a remedy, at least the insufficient air handling unit has to be replaced by an air handling unit suited for destroying chemical and especially microbiological contaminants. Said replacement works may be complicated since in many cases the new air handling unit will be much more voluminous compared with the used insufficient air handling unit. Therefore, in many cases insufficient air handling units themselves or insufficient mechanical filters in air handling units are not replaced with the result of risks for the health of the humans or animals within the room. The term "room" is used here including rooms within private houses, meeting rooms, business sites, conference centers or similar, factory halls, stables and so on.

Air handling units which are suitable for destroying airborne contaminants often include Advanced Oxidation Process (AOP), such as catalysts, and are used in air conditioning systems or air purifying systems for cleaning ambient air in hospitals, quarantine stations or public places like restaurants and the like. It is also known to use a combination of more than one catalyst, mainly a catalyst having an activity initiated by electromagnetic radiation, especially UV radiation, and a further catalyst which may be effective already at ambient temperature. The combination of both catalysts is found effective to destroy microbiological pollutions by the catalyst activated by means of electromagnetic radiation and to completely mineralize the reaction products of the first catalyst by means of the second catalyst. Examples of respective catalysts are described in CN 106582265 A and JP H11-137656 A. As an electromagnetic radiation source UV lamps are used and have to be arranged near the respective catalyst.

It is an object of the present invention to allow the depollution of air within closed rooms in combination with present air conditioning installations.

According to the present invention an insert device as mentioned above has a frame casing with an outer closed periphery shaped to be mounted within the predetermined cross section of the casing of the air handling unit and having end faces open for the air flow, an air permeable catalytic grid structure comprising a carrier grid and a coating with a catalytic material is held at each of the end faces, the catalytic material is a mixture comprising an adsorbent, a first catalyst activatable by electromagnetic radiation and a second catalyst being activated at low temperature, and the frame casing in addition holds an electromagnetic radiation source device between the grid structures at its end faces.

The present invention is based on the idea to provide the air handling necessary for the destruction of chemical and biological contaminants within a compact insert device which can be inserted into commercially available air handling units of air conditioning installations in place of or in addition to a filter of said air handling unit. Consequently, the present invention allows an easy upgrade of customary air conditioning installations so that they become effective to destroy chemical and biological contaminants without severe reconstructions of the air conditioning installation. The second catalyst is already activated at low temperature which is especially the ambient temperature so that the second catalyst can be effective at ambient temperature even without a specific activator. Low temperature is meant to be below 70°C, especially below 50°C.

For many purposes it is advantageous to include a fan causing the air flow in the air handling unit. In some embodiments the air handling unit is a part of a fixed installation and connected to an air guiding tubing. In other embodiments the air handling unit is a stand-alone unit or a cabinet connected to at least a tubing for sucking air to be purified and/or for distributing decontaminated air to desired place(s).

The insert device of the present invention may be designed to fit within a frame construction of the air handling unit designed for holding one or more filter units. That means that according to the present invention a frame construction as known for customary filters can be used for holding the insert device comprising the complete arrangement for destroying chemical and biological contaminants.

According to an aspect of the present invention an air conditioning installation comprising an air handling unit guiding air flowing in a given flow direction wherein the air handling unit has a casing with a predetermined cross section in which at least a filter is housed, the filter being held by a support frame having a peripheral shape adapted to the free cross section of the air handling unit is characterized in that the insert device is sealingly inserted into the support frame. Within the air handling unit the insert device with the catalysts may establish a filter unit which can be arranged and used in place of a previous filter unit or in addition to one or more other further units which may be usual filter units like absorbents. "sealingly inserted" means that there is no considerable bypass for the air flowing in the air handling unit so that essentially all of the air will pass through the insert device. A perfect seal is not required. In a specific embodiment the air handling unit also includes a fan for causing the air flow.

Since the customary filter units are arranged so as to be easily removable from the air handling unit for cleaning and/or replacement purposes the insert device according to the present invention can be easily inserted into the air handling unit.

Accordingly, the insert device of the present invention allows an easy upgrade of customary air conditioning installations without using additional space or additional cabinets or similar.

In an embodiment of the insert device of the present invention the first catalyst is activatable by UV radiation and the electromagnetic radiation source emits UV radiation. For this purpose at least one UV source may be arranged as the electromagnetic radiation source device between the grid structures. The UV source may be at least a conventional UV lamp as well as an arrangement of UV LEDs.

In another embodiment the electromagnetic radiation source device may be a plasma generator which generates UV radiation due to the radiation of a plasma which is preferably a cold plasma. In this embodiment the effect of the catalysts may be enhanced by means of the plasma wherein highly reactive species are formed which are able to destroy chemical as well as biological contaminants.

When both catalysts as well as the absorber are each mixed in a pulverulent condition a very fine distribution of the respective particles can be obtained when the grid carrier is coated with a slurry of said pulverulent materials.

In an embodiment of the present invention the catalytic grid structure is inert to the oxido-reduction process. It may have a honeycomb structure which provides a sufficient stability and a large surface for the catalytic effect combined with a low resistance for the flow of the air through the grid structure.

If necessary, the catalytic grid structure may be completely covered by a layer of an air permeable flexible filter material on the respective outer side of the insert device. That optional filter material may be especially designed to withhold UV radiation within the insert device so that no UV radiation will reach the outside region of the insert device and possibly harm a person working near the air handling unit. That layer may have a thickness of about 1 mm and can consist of a non-woven, e. g. from fiber glass. In another embodiment said layer may be in metallic grid having a mesh width in the order of some µm so as to withhold UV radiation on one hand and cause a low pressure drop for the flowing air on the other hand. For this purpose very fine walls of the metallic grid are necessary leading to a high flexibility of said layers at the input and the output side of the insert device establishing the catalytic reactor. For a mechanical stabilization the layers may be held by a support grid with a comparatively lower flexibility but with a mesh width of some millimeters so as to not increase the pressure drop for the flowing air.

The invention will be described in more detail with reference to embodiments illustrated in the accompanying drawings.
- Figure 1: - is a side view on parts of an air conditioning installation with an air handling unit shown in a sectional view,
- Figure 2: - is a partly exploded view of a first embodiment of an inserted device,
- Figure 3: - is a perspective view of a mounted insert device,
- Figure 4: - shows a detail of figure 3 in a perspective view,
- Figure 5: - is a long longitudinal sectional view of the complete insert device,
- Figure 6: - is a transversal sectional view of the inserted device,
- Figure 7: - is a schematic perspective view showing interior walls of an air handling unit with a standard support frame which can carry the insert device,
- Figure 8: - shows the insert device mounted into the support frame,
- Figure 9: - shows a different embodiment of a support frame designed for carrying four filter units,
- Figure 10: - shows four insert devices mounted into the frame of figure 9,
- Figure 11: - is an exploded view of partly optional components of a grid structure,
- Figure 12: - is a sectional view of the grid structure shown in figure 11,
- Figure 13: - is a perspective view of an embodiment of an air handling unit within a rack cabinet.

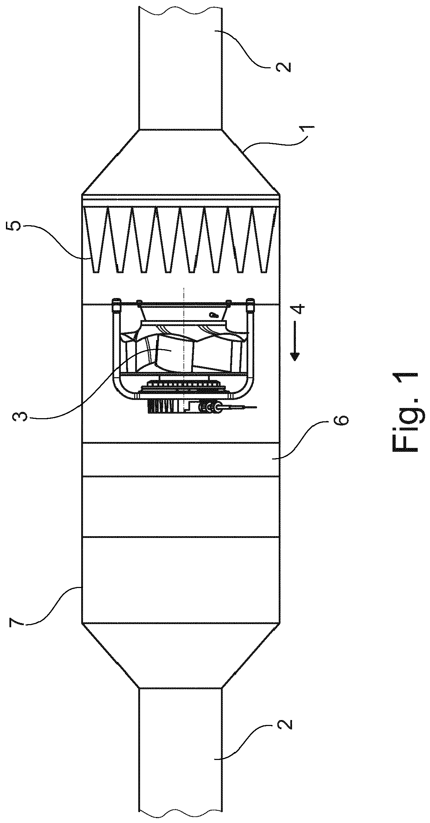

Figure 1 shows an essential part of an air conditioning installation consisting of an air handling unit 1 to which an air guiding tubing 2 is connected at both ends. In the embodiment of figure 1 the effective or maximum diameter of the air handling unit 1 is somewhat larger than the diameter of the air guiding tubing 2.

Within the air handling unit 1 a fan 3 is positioned which serves for moving the air within the air handling unit 1 and the air guiding tubing 2 in a predetermined flow direction 4 indicated by an arrow in figure 1. The air guiding tubing 2 will guide the air driven by fan 3 into an essentially closed room (not shown) as purified air and will suck off the room air and guide it to the air handling unit 1 for filtering and purification.

The air handling unit 1 has conical ends for establishing the transition from the smaller diameter of the air guiding tubing 2 to the larger dimensions of the air handling unit 1. The air flowing into the air handling unit 1 passes a pre-filter 5 which regularly is a coarse filter for withholding larger particles of the air stream. Thereafter the air is accelerated by the fan 3 and then passes an insert device 6 according to the present invention. It can be seen from figure 1 that the insert device 6 extends over the whole free diameter of the air handling unit 1 so that the complete air flow will pass through the insert device 6.

After passing the insert device 6 the air flow may furthermore pass through a filter unit 7 which can optionally be arranged at the downstream end of the air handling unit 1. The filter unit 7 may consist of or comprise a HEPA (high efficiency particulate air) filter which is customary for air conditioning installations.

Figure 1 shows that the insert device according to the present invention is arranged within the air handling unit 1 in the same way as the pre-filter 5 and the filter unit 7 so that it can be easily mounted within the air handling unit 1 and will not need extra space within the air conditioning installation. Although figure 1 showing a fan 3 within the air handing unit 1 the fan 3 for causing the air flow may also be positioned at other places within the air circuit, such as within the air guiding tubing 2 or at an entrance of the air guiding tubing 2.

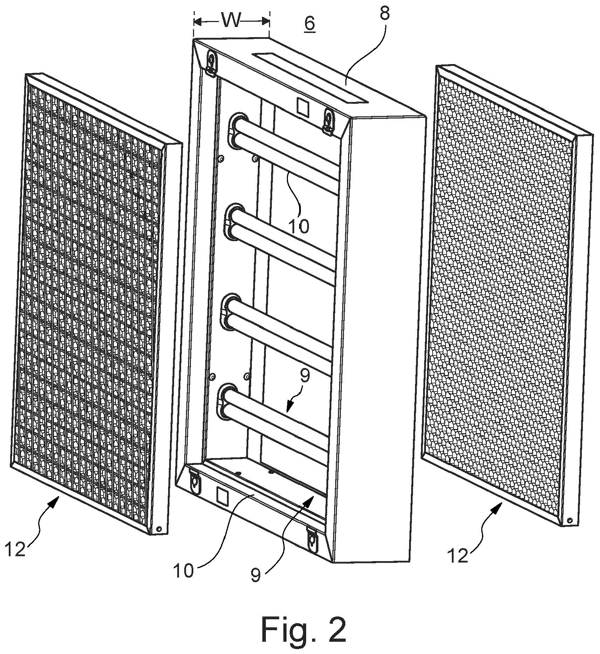

Figure 2 shows an embodiment of an insert device 6 the basic structure of which allows the insertion in the air handling unit 1. The insert device 6 consists of a frame casing 8 which forms an outer closed periphery with two open end faces 9. The frame casing 8 has a width W which is smaller than the dimensions of its height and depth, each. In this embodiment the frame casing 8 holds in its interior eight UV lamps 10 which are arranged in pairs with equal distances between said pairs over the height of the frame casing 8. The height and the depth of the frame casing 8 match with the respective dimensions of the free section of the air handling unit 1 as will be described later in more detail. The frame casing 8 forms circumferential seats 11 wherein catalytic grid structures 12 are inserted on both open end faces 9 in order to close the case for the insert device 6. The catalytic grid structures 12 are air permeable so that they will not cause an unreasonable pressure drop of the air stream in its flow direction. From the drawings it is clear that the frame casing 8 is oriented with its width parallel to the flow direction 4.

The shape of the frame casing 8 as well as the catalytic grid structures 12 depend on the dimensions of the free section of the air handling unit 1 of the air conditioning installation.

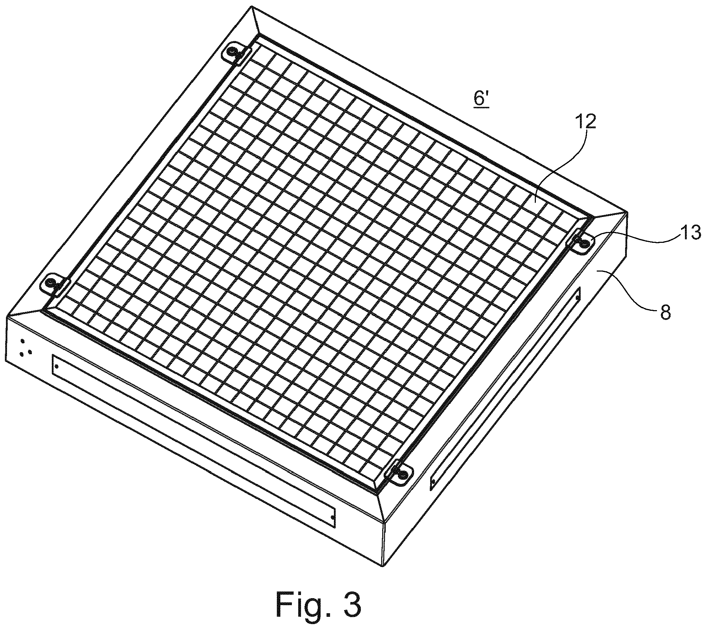

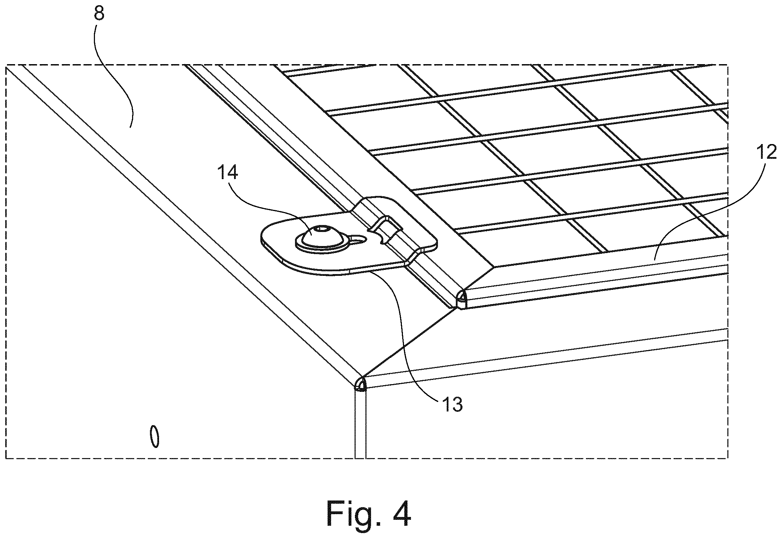

Figure 3 shows a perspective view of another embodiment of an insert device 6' which is designed for a square section of an air handling unit 1. The insert device 6' is shown in a mounted state where the catalytic grid structures 12 are inserted into the respective seats 11 of the frame casing 8 and are held in position by means of brackets 13 which hold the catalytic grid structure 12 in position without extending into the flow path of the air circulated or driven by the fan 3. The bracket is mounted to the frame casing 8 by means of a screw 14. This is shown in detail in figure 4. The surface of the catalytic grid structure 12 is at least nearly flush with the surface of the frame casing 8 and may protrude from this surface only a little bit.

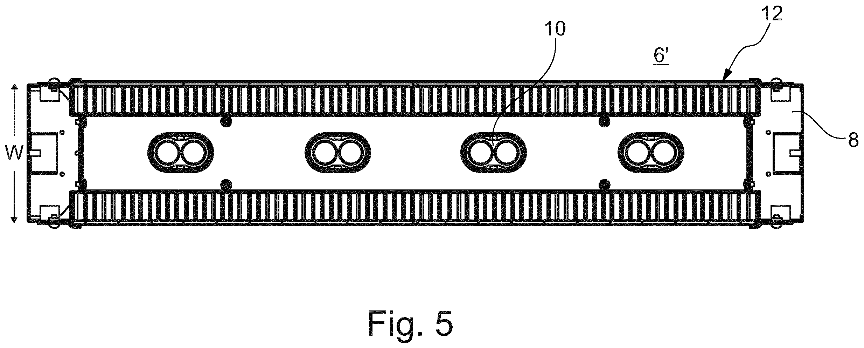

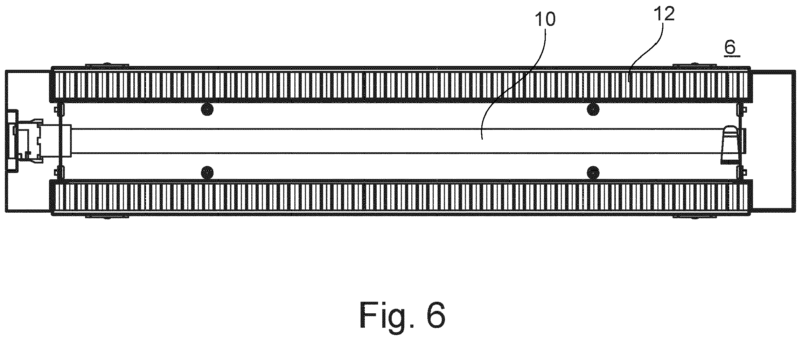

Figures 5 and 6 show two cross sectional views of the mounted insert device 6' in two directions perpendicular to each other. The frame casing 8 extends along periphery with the width W. The open end faces 9 are closed by the catalytic grid structures 12 which are described later in detail. In the interior of the casing of the insert device 6' the UV lamps 10 are held and serve for activating a photocatalytic component of the catalytic grid structures 12, as will be explained in detail below.

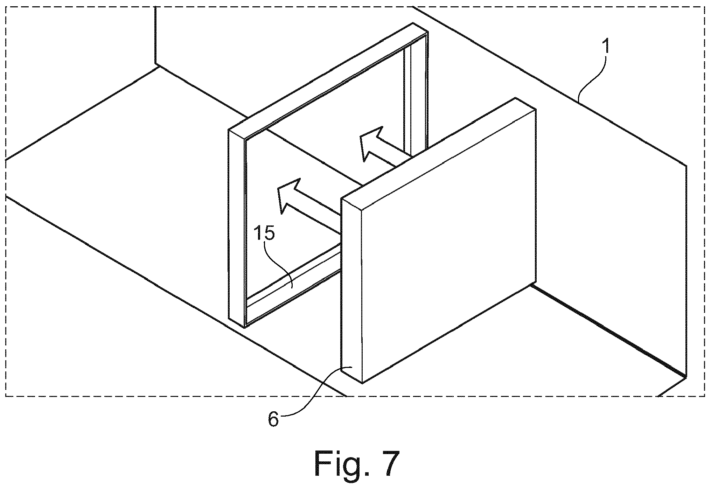

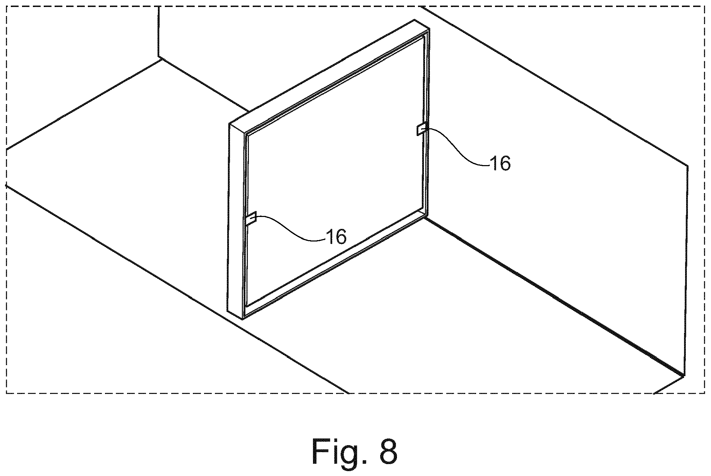

Figures 7 and 8 show a schematic view into the interior of the air handling unit which forms a rectangular channel. There is used and fixed a standard support frame 15 for filter units. The inserted device 6 is designed and dimensioned so as to fit to said standard support frame 15 so that the surfaces of the inserted device 6 are essentially flush with the standard support frame 15. Therefore, the fixation of the insert device 6 within the standard support frame 15 can easily be performed by brackets 16.

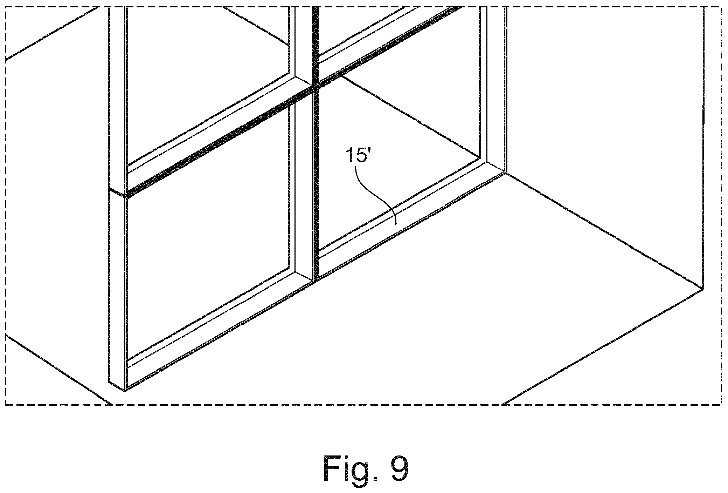

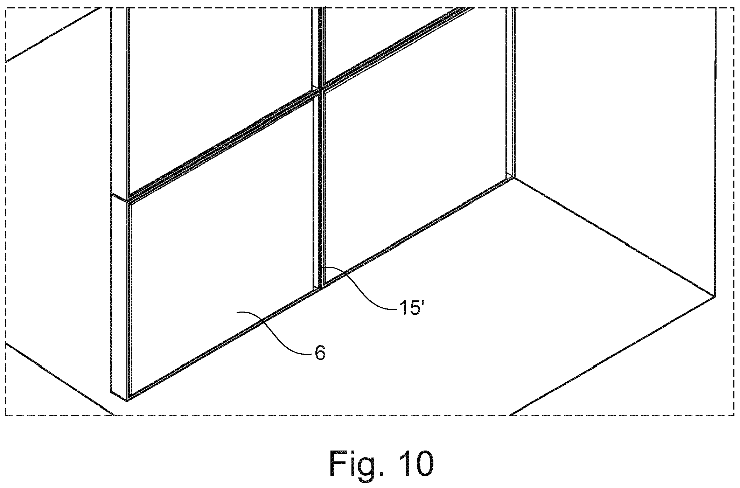

Figures 9 and 10 show an embodiment where the standard support frame 15' provides four seats for filter units. This embodiment serves for dealing with greater free cross sections of the air handling unit 1. The standard support frame 15' may hold four filter units and provide a higher stability compared with a larger frame holding only one filter unit. This modified support frame 15' can be used for holding four insert devices 6 as illustrated in figure 10.

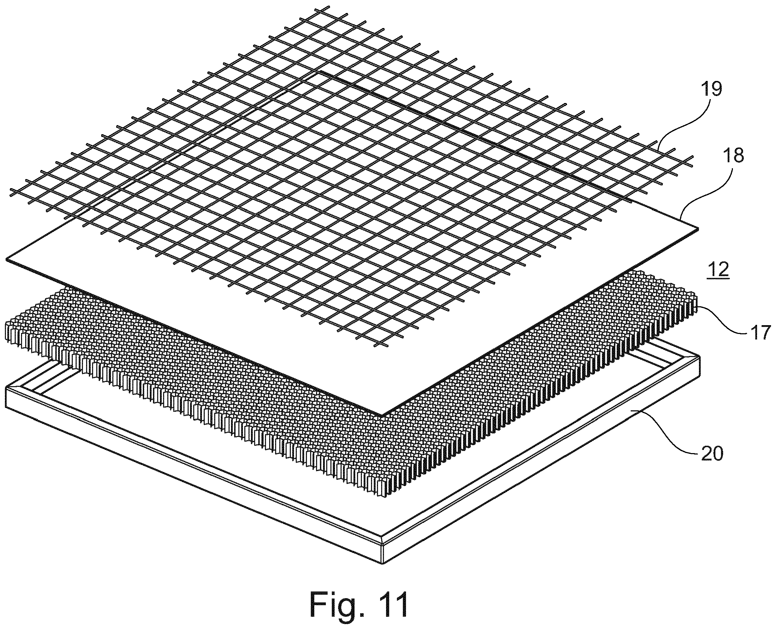

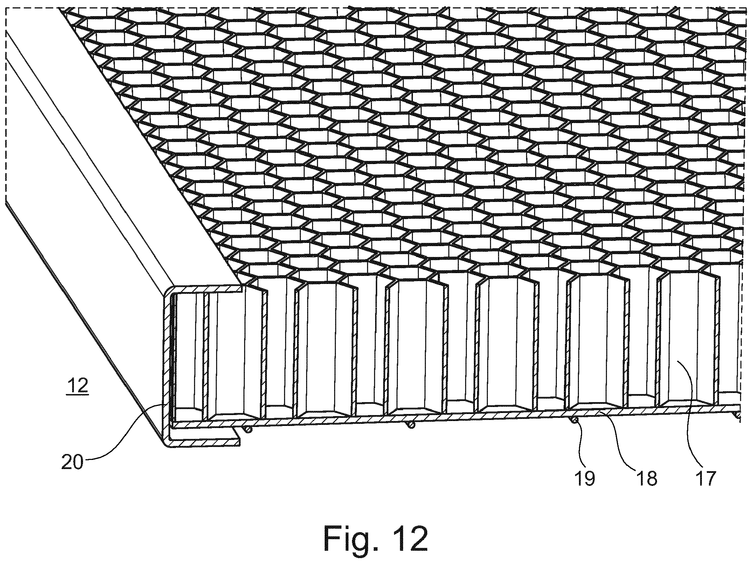

An embodiment of the catalytic grid structure 12 is illustrated in figures 11 and 12. The essential element of the catalytic grid structure 12 is a carrier grid 17 consistent e. g. of aluminum. The carrier grid 17 may have a honeycomb structure so as to provide hexagonal channels through which the air flow must pass. Other carrier grid structures like rectangular, square or diamond or rhomb grids may be used. The material of the carrier grid 17 can be chosen in any suitable way and may e. g. be an other metal or a suitable plastic material. The carrier grid 17 is coated with catalytic material. The compact structure of the insert device 6, 6' is achieved due to the highly efficient catalytic material consisting of a first catalyst, a second catalyst and in adsorbent, said components are produced separately and are mixed in pulverulent condition each. The mixture is put in a suitable liquid e. g. a mixture of water and alcohol, to form a slurry which can then be coated on the whole surface of the carrier grid 17. The coating preferably takes place in several steps with thin layers each which together form the uniform coating of the desired thickness which may be between 1 µm and 250 µm. The coating may be performed by various methods including dip coating or spray coating. After each coating step the thin layer is allowed to dry before the next layer is applied. Thereby a binder-free coating can be achieved enhancing the efficiency of the catalyst.

The through holes or channels of the carrier grid 17 are designed so as to only little reduce the free cross section of the air handling unit 1 when the insert device 6 is mounted in the air handling unit 1. The area of the through hole may account for at least 80 % preferably at least 90 % of the area of the free cross section of the air handling unit 1. In order to provide a sufficient catalytic effect the length of the through holes should be at least 5 mm, in other embodiments at least 10 mm, 15 mm or 20 mm, up to 50 mm and more. The ratio of the length of the through holes and their distance between limiting walls ("diameter") is preferably larger than 2, in other embodiments larger than 5, larger than 10 and in these cases not larger than 50, in other embodiments not larger than 30 or not larger than 20. The distance of the limiting walls of the through holes (for round through holes the diameter) is at least 2 mm, in other embodiments at least 5 mm or at least 7 mm. An upper limit is 50 mm, in other embodiments 20 mm or 10 mm. A straight channel formed by the through holes is preferred in view of a minimum pressure drop caused by the grid structure.

Figure 11 shows a further layer 18 which extends over the whole area of the carrier grid 17. Said further layer 18 is an air-permeable thin flexible layer which may have filter purposes and especially be able to block UV radiation. The thickness of this layer may be about 1 mm and not exceed 2 mm. It may consist of a non-woven e. g. from fiber glass, or may be a metal grid having a mesh width in the order of some µm, e. g. between 1 and 10 µm. Said layer is positioned on the surface of the carrier grid extending away from the UV lamps 10 in the insert device 6.

Since the further layer 18 may be very thin and flexible, a stabilization by a wire grid 19 is advisable. The wire grid is for stabilization purposes only and therefore may have a large mesh width, e. g. between 5 and 15 mm with a wire diameter between 0,2 and 1,0 mm.

The sandwich arrangement of carrier grid 17, further layer 18 and wire grid 19 can preferably be mounted in a holding frame 20 so that the catalytic grid structure 12 can easily be handled as a unit, even if a further layer 18 and a wire grid 19 is used. However, it should be noted, that the further layer 18 and the wire grid 19 are optional and may be omitted in some applications. The completely mounted catalytic grid structure 12 according to figure 11 is represented in an enlarged sectional view in figure 12.

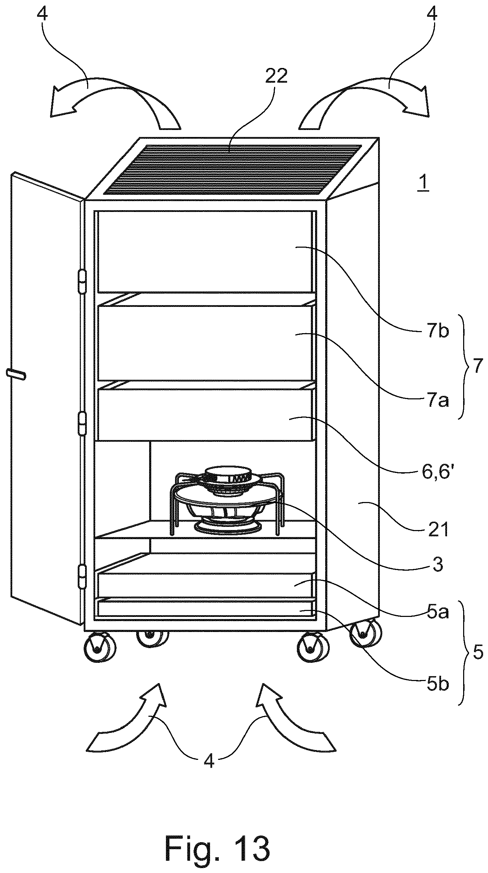

Figure 13 shows an embodiment of a mobile air handling unit 1 in form a rack cabinet 21 through which air may flow from the bottom to the top as indicated by arrows representing the flow direction 4 of the air. After passing an air permeable bottom of the rack cabinet 21 the air flows through a pre-filter 5 consisting of a coarse pre-filter 5a and a fine pre-filter 5b. Above the pre-filter 5 the fan is positioned so that air is sucked through the pre-filter 5 and afterwards pressed through downstream units. The first downstream unit in this embodiment is the insert device 6, 6' as described hereinbefore. Downstream a filter unit 7 follows which may consist of an absorbent filter 7a if specific gases are being removed, and a HEPA (high efficiency particulate air) filter 7b, if necessary. The topside of the rack cabinet 21 is provided with fins 22 through which the cleaned air is distributed into the ambient room. The fins 22 may be adjustable thereby forming adjustable outlet slots.

The compact structure of the insert device 6 is especially possible with a special coating material for the carrier grid 17.

The coating preferably has a total thickness of at least 50 µm, preferably at least 75 µm, especially at least 100 µm. A coating of such thickness especially provides a satisfactory amount of catalyst for gas depollution. Preferably the thickness does not exceed 500 µm, more preferably does not exceed 350 µm, and especially does not exceed 250 µm. A preferred range lies between 100 µm and 250 µm. If the coating is too thick, it is possible that lower layers are not sufficiently irradiated and/or not sufficiently reached by contaminants to be depolluted. It can therefore lead to a waste of catalytic material.

The first catalyst can for example consist of tungsten oxide and/or zinc oxide. It is preferred but not necessarily the case that only one type of first catalyst is used. It is also possible to use more than one catalyst having photocatalytic activity.

In a preferred embodiment the first catalyst is titanium dioxide TiO₂. Titanium dioxide is very well known for its photocatalytic properties. It is a semiconductor and can be activated by irradiation with UV and/or visible radiation of a wavelength from 100 nm to 400 nm. Preferably the UV radiation used for activating the first catalyst, especially titanium dioxide, has a wavelength of 365 nm or less, more preferable in the UV-C range between 100 nm and 280 nm, most preferably of 254 nm. The use of UV radiation in the UV-C range has the advantage of additionally using the direct disinfecting properties of the UV-C radiation. UV-C radiation therefore acts directly biocidal due to its high energy and indirectly biocidal as well as on non-biological contaminants by activating the first catalyst. The titanium dioxide can be already photo-activated.

For the activation of the first catalyst, preferably any kind of UV radiation emitting sources or light sources can be used, for example artificial lamps emitting UV radiation or light emitting diodes or fluorescent tubes emitting UV radiation or UV radiation formed by cold plasma-type electrodes.

Titanium dioxide has the advantage of being cost-efficient and having the ability to at least partly regenerate itself. It is highly active against different contaminants, including chemical and biological contaminants.

It is known that titanium dioxide in the crystalline form of anatase has the highest catalytic activity. Therefore it is one embodiment of the invention that the titanium dioxide completely is of the anatase type.

Explorations of the applicant on the other hand have shown that a certain proportion of titanium dioxide of the rutile type, against the prevailing opinion, is enhancing the overall depolluting capacities of the catalytic device. The separation of the electric charges on the surface of the catalyst is augmented and so is its efficiency.

It is a preferred embodiment of the invention that the first catalyst is titanium dioxide TiO₂ in the form of a mixture of anatase and rutile with an anatase/rutile ratio between 60/40 and 99/1. Preferably the ratio is at least 60/40, more preferably at least 70/30 and especially at least 80/20. The ratio is preferably not exceeding 99/1, more preferably not exceeding 95/5, and especially not exceeding 90/10. In a further preferred embodiment the ratio is between 77/23 and 83/17, especially 80/20.

In a preferred embodiment the first catalyst, especially titanium dioxide, is doped, especially with silver ions or platinum ions. Doping of the first catalyst increases the biocidal effect on biological contaminants and on the destruction of possibly harmful byproducts of the destroyed contaminants. The presence of the doping ions increases the number of possible oxidation and reduction reactions.

In a preferred embodiment the second catalyst is a low-temperature catalyst. A low temperature catalyst preferably is a catalyst being already catalytically active at temperature lower than 100 °C, more preferably at temperatures lower than 50 °C, most preferably already at room temperature of 20 °C. This does however not imply, that it must be inactive at higher temperatures. Preferably the catalytic activity is increased with increasing temperature at least over a certain temperature interval, preferably 20°C to 100 °C or 50° C to 100 °C.

Low-temperature catalysts are for example metal oxides like nickel oxide or cerium oxide. In a preferred embodiment of the invention the second catalyst is manganese monoxide MnO, which is an efficient low-temperature catalyst. Explorations of the applicants have shown that manganese monoxide is significantly more efficient than the well-known manganese dioxide MnO₂, whose catalytic activity is insufficient. Therefore preferably the manganese monoxide used in this embodiment is not or substantially not contaminated with manganese dioxide. In a preferred embodiment, the amount of manganese dioxide is lower than 5 %, more preferably lower than 1 %, most preferably lower than 0.1 % of the total mass of the manganese monoxide used as second catalyst. Optimally, no manganese dioxide is present as an impurity of the manganese monoxide second catalyst.

Manganese monoxide, especially in crystalline form, allows the generation of highly reactive radical species when in contact with oxygen, for example from the air. Preferably the temperature is higher than 35 °C, especially between 35 °C and 55 °C, more preferably between 45 and 50 °C. Preferably the relative humidity of the gas to be depolluted, especially air, is between 30 - 80 %, more preferably 50 %. Under these circumstances a very efficient generation of the radical species is possible. The radical species are able to react also with very small contaminants in the nano or micro scale, such as aldehydes like formaldehyde, which are usually hard to crack. This is, inter alia, because such small molecules are hard to be trapped. Hydrophilic catalysts are usually quickly saturated with water or other polar small molecules so that hardly any sites are available for small contaminants to be trapped and then oxidized. Manganese monoxide offers the specific advantage that it is only poorly reacting with water and trapping water molecules on its surface so that more sites are available for contaminants. Furthermore, the pores within the manganese monoxide are preferably smaller than those of the titanium dioxide so that larger molecules are trapped less and, again, more sites remain available for small contaminants. The radical species also react with biological contaminants.

A disadvantage of manganese monoxide is that it is unstable compared to manganese dioxide. Therefore, when synthesizing manganese monoxide, special care needs to be taken on the reaction parameters, when the formation of manganese dioxide is to be avoided, as preferred. One important reaction parameter is the temperature. When using a calcination temperature higher than 300 °C, a substantial amount or solely manganese dioxide is formed.

Since the formation of titanium dioxide from precursors usually requires temperatures of more than 300 °C, for example 300° C - 600 °C, a simultaneous calcination of manganese monoxide precursors and titanium dioxide precursors to form manganese monoxide on the one hand and titanium dioxide on the other hand, is impossible. A calcination temperature of higher than 600 °C during the formation of the titanium dioxide may lead to an excessive formation of rutile, which is undesired. On the other hand, a certain amount of rutile, as described above, enhances the efficiency of the catalyst.

Therefore, when the first catalyst is titanium dioxide and the second catalyst is manganese monoxide, preferably no simultaneous calcination takes place.

It is preferred but not necessarily the case, that the synthesis of the first catalyst, the second catalyst and/or the adsorbent is part of the manufacturing method of the catalytic device. Preferred embodiments of the synthesis will be explained in detail below. If at least the synthesis of titanium dioxide as first catalyst and manganese monoxide as second catalyst is part of the manufacturing method, no simultaneous calcination of their precursors preferably takes place. They are preferably synthesized apart from each other.

One advantage of using a low-temperature catalyst, especially manganese monoxide, is that the necessarily accruing waste heat of the UV radiation source can be used to warm the low-temperature catalyst thereby increasing its catalytic efficiency. Hence, a synergy can be achieved when combining a photocatalyst and a low-temperature catalyst in one stage and preferably arrange them spatially close to the UV radiation source in order to use the waste heat efficiently. Preferably a temperature of up to 40 °C, up to 50 °C or up to 90 °C can be achieved on the surface of the catalytic device.

The adsorbent is preferably a compound having a large specific surface area, preferably of at least 300 m²/g, more preferably of at least 500 m²/g, most preferably of at least 1000 m²/g, especially more than 2000 m²/g. The adsorbent can for example be activated carbon or activated coke.

In a preferred embodiment the adsorbent is a zeolite. Zeolites are microporous aluminosilicate minerals which can be naturally occurring or artificial. Zeolites can be synthetic.

Preferably a hydrophilic zeolite is used, as explorations of the applicant show that biological contaminants tend to be better adsorbed by a hydrophilic than a hydrophobic zeolite. Preferably a zeolite of type A or ZSM-5 is used. The zeolite is preferably a synthetic zeolite, especially a synthetic zeolite of type A or ZSM-5. Synthetic zeolite has the advantage of being pure and having a homogenous structure due to the fact that is synthesized.

In a preferred embodiment of the invention, the first catalyst is titanium dioxide, the second catalyst is manganese monoxide and/or the adsorbent is a zeolite. The zeolite is preferably a synthetic hydrophilic zeolite of type A. These configurations are preferred for all applicable embodiments described herein.

Hydrophilic zeolite of type A has a high affinity to chemical and biological contaminants and no natural equivalent. It has an affinity to the cellular membranes of microorganisms. These adsorb to the surface of the zeolite for electrostatic reasons. Additionally, the hydrophilic zeolite of type A has a direct antimicrobial effect, which makes its use even more synergistic. Hydrophilic zeolite of type A has a crystal structure formed from an anionic aluminosilicate structure, neutralized by alkaline or alkaline earth metal cations.

Preferably the zeolite, especially the zeolite of type A comprises a sodalite crystal structure.

The structure of the zeolite links multiple elementary sodalite cages. A sodalite cage consists of multiple polyhedrons with eight hexagonal faces and six square-shaped faces. The sodalite cages forming the zeolite are interconnected via the square-shaped faces. The specific structure of the sodalite cages gives the zeolite an open 3D structure, whereby especially 47 % of the total volume is formed by interstices. Therefore the zeolite provides a large surface for adsorption of biological and chemical contaminants within a small volume. Additionally the open 3D structure allows for a high water absorption and retention capacity. Since water can be used to form highly reactive radical species like the hydroxyl radical on the surface of the catalysts, this can be advantageous. Furthermore the water retention capacity may, depending on the circumstances, ensure that no water film is formed on the catalysts, thereby degrading their efficiency. On the other hand a very high amount of adsorbent, especially zeolite, may provide for a water excess, thereby degrading the efficiency of the catalysts. Preferably the amount of adsorbent therefore does not exceed 80 %, especially does not exceed 70 % of the total mass of first catalyst, second catalyst and adsorbent. On the other hand, if the amount of adsorbent is very low, the water retention capacity might not be sufficient. Therefore the amount of adsorbent preferably is not lower than 40 % especially not lower than 30 % of the total mass of first catalyst, second catalyst and adsorbent.

The inventors have explored that the combination of two different catalysts and an adsorbent is synergistically effective for depolluting a gas. The contaminants are adsorbed onto the adsorbent. By way of the mass transfer phenomenon, they migrate to the catalyst particles. The generation of reactive species on the surface of the catalysts then leads to destruction of the contaminants, preferably to complete mineralization of the contaminant. Also byproducts during the destruction of the contaminants are preferably destroyed further so that no possibly harmful byproducts are released into the depolluted gas or are released but destroyed within one of the next depolluting cycles. The adsorbent can thereby also form a reservoir for pollutants to be destroyed, for example during a peak load of the gas with contaminants. An amount of contaminants that would exceed the catalytic capacity of the catalysts per time unit can therefore preferably be held back by the adsorbent and then be guided to the catalysts with a time delay. This makes it possible to also treat peak loads that would, without adsorbent, exceed the capacity of the catalysts per se. In order to provide good adsorbing properties, the amount of the adsorbent is preferably higher than the amount of each catalyst and/or the cumulated amount of the catalysts, with respect of the total mass of the first catalyst, the second catalyst and the adsorbent.

The adsorbent is holding back contaminants which are then transferred to the catalysts via mass transfer. On the other side, by this phenomenon, the adsorbent is constantly regenerated and its adsorbing capacity restored. This demonstrates a real synergy of providing these compounds within one mixture. In order to optimally achieve this effect, the mingling of the catalysts and the adsorbent is preferably performed intensively, especially to provide a mixture as homogenous as possible.

The amount of the first catalyst is preferably at least 10 %, more preferably at least 20% and does preferably not exceed 30 %, especially does not exceed 40 % of the total mass of the first catalyst, the second catalyst and the adsorbent. The amount of the second catalyst is preferably at least 5 %, more preferably at least 10 %, especially at least 15 % and does preferably not exceed 20 %, especially does not exceed 30 % of the total mass of the first catalyst, the second catalyst and the adsorbent.

In a preferred embodiment, the components are provided in the following ranges, in weight percent with regard to their total mass: Between 27% and 30% of the first catalyst, between 11% and 17% of the second catalyst and between 55% and 59% of the adsorbent.

A specific and preferred embodiment contains an amount of 29 % of the first catalyst, 12 % of the second catalyst and 59 % of the adsorbent.

The explorations of the applicants showed that this ratio shows a good efficiency as well as satisfying water retention capacities and contaminant retention capacities. With these amounts an especially sustainable and long lasting composition is provided that also provides very good depolluting attributes. Additionally, the amount of UV/visible radiation needed to activate the first catalyst provides for a sufficient and useful amount of waste heat to increase the catalytic activity of the second catalyst in this amount, when the second catalyst is a low-temperature catalyst.

Additionally, this invention relates to an embodiment in which the amount of the first catalyst is lower than 27 % and higher than 30 %, the amount of the second catalyst is lower than 11 % and higher than 17 % and the amount of the adsorbent is lower than 55% and higher than 59%, each in weight percent and with regard to their total mass. All further preferred configurations and embodiments described herein apply to this specific embodiment.

As stated above it is preferred but not necessary, that the synthesis of the first catalyst, the second catalyst and/or the adsorbent is part of the manufacturing method of the catalytic device. The syntheses each are performed apart from another.

If the first catalyst, as preferred, is titanium dioxide, it is preferably synthesized by using a sol-gel process and precursors like titanium tetrachloride or butyl titanate, followed by a calcination at temperatures of 300 - 600 °C. Also different methods for producing powdered titanium dioxide like thermal plasma technology, laser pyrolysis, hydrothermal or electrochemical synthesis are possible.

A preferred sol-gel process for synthesizing the titanium dioxide first catalyst in a powdered form is now described in detail:
The precursor titanium tetrachloride is mixed with absolute ethanol in a ratio between 0.5:10 and 3:10, preferably with a ratio of 1:10, for 4h at room temperature. The formed sol is then placed in an ultrasonic bath for a time of 20 to 40 minutes, preferably 30 minutes. Consecutively the sol is dried at a temperature of 100 °C and 130 °C, preferably 120 °C for a time between 1h and 24h, preferably 7h in order to obtain a powder. This powder is then progressively calcined at a temperature between 530 and 570 °C, preferably at 550 °C, for 2h to 3h, preferably 2h. Thereby the temperature is increased in steps of 10 - 12 °C/minute. The obtained powdered titanium oxide is then cleansed with deionized water and consecutively dried at a temperature between 90 and 110 °C, preferably 100 °C.

If the second catalyst is, as preferred, manganese monoxide, it is preferably synthesized from precursors like manganese acetate, manganese nitrate and/or manganese sulfate followed by a calcination at temperatures below 300 °C.

A preferred process for synthesizing the manganese monoxide second catalyst in a powdered form is now described in detail:
A precursor, preferably manganese acetate, is mixed with a solution of potassium permanganate in a ratio in mol/I of 1,8/1,2 for at least 24 h at room temperature between 21 and 25 °C. Afterwards it is filtered and rinsed with deionized water. The obtained powder is calcined for several hours, preferably 72h, with an increase in temperature of 6°C/min until a temperature between 280 and 300 °C is reached. The result is rinsed with deionized water and consecutively dried at 100°C until the powdered manganese monoxide is obtained which can then be used. In order to obtain a well useable powder, the liquid is preferably fully evaporated.

The low temperature below 300 °C is used to avoid the formation of the more stable but undesired manganese dioxide.

If the adsorbent is a synthetic hydrophilic zeolite of type A, it is preferably synthesized as follows:
Step 1: Providing a sodium hydroxide solution by mixing of sodium hydroxide, for example pellets of sodium hydroxide, in distilled water with a ratio of 110:1 to obtain a homogenous sodium hydroxide solution.
Step 2: Dividing the sodium hydroxide solution into two equal parts, thereby obtaining a first volume and a second volume of sodium hydroxide solution.
Step 3: Crystalline odium aluminate is, volume per volume and within 10 to 20 min, mixed to the first volume up to 17 % to obtain a solution A.
Step 4: Crystalline sodium silicate Na₂SiO₃ is, volume per volume, solved in distilled water up to 57 % to obtain a solution B.
Step 5: Solution B, is mixed with the second volume, with a ratio of 3.8/1000, to obtain a mixture C, preferably a clear mixture C.
Step 6: The clear mixture C is added to solution A to obtain a mixture D.
Step 7: Mixture D is heated until the water is fully evaporated.
Step 8: Mixture D is cooled by lowering the temperature until a solid E appears.
Step 9: The solid E is filtered and rinsed with distilled water until a pH-value between 8.5 and 9.5 of the rinsing water is obtained.
Step 10: Drying the solid E for a period of 8h to 15h, preferably 12h, at a temperature between 100 and 120 °C, preferably 110 °C, to obtain the synthetic hydrophilic zeolite of type A.
Step 11: Grinding the synthetic hydrophilic zeolite of type A into a powder.

Preferably, in step 7, the temperature is between 75 and 130 °C, preferably 100 °C for 3h to 4h. It is the target to fully evaporate the water from mixture D.

Preferably, in step 8, mixture D is cooled by lowering the temperature to a value lower than in step 7 but above room temperature. This cooling allows to avoid the forming crystals of solid E to stick to the bottom of the container in which mixture D is initially located. In one specific embodiment of step 8, the temperature is lowered to 30 °C, considering that room temperature is between 20 and 25 °C, so that it is easy to remove solid E without it sticking to the bottom.

In a usual manner the pH-value of the rinsing water in step 9 is measured with a pH meter or any other measuring device known by the person skilled in the art that allow a determination of the pH-value. By measuring the pH value of the rinsing water indirectly the surface pH-value of solid E, which will form the synthetic hydrophilic zeolite of type A is measured.

It is preferred that the surface of the zeolite is slightly alkaline, since this enhances the formation of van der Waals forces with the titanium dioxide and the manganese monoxide, which have a slightly acidic surface. This facilitates to generate a homogenous catalytic composition.

In step 10 the drying duration and temperature of solid E allow a gradual removal of the water molecules on the surface of the zeolite. Removing the water too rapidly would lead to the risk of crack formation in the surface of the zeolite. This cracks would weaken the important 3D structure. This is why a drying time between 8 and 15 h with a temperature of 100 - 120 °C is chosen, to progressively remove the water molecules without risking to weaken the structure of the zeolite.

In step 11 the synthetic hydrophilic zeolite of type A can be grinded using any grinding device.

In a particular embodiment, the adsorbent, especially the zeolite, is grinded to achieve a particle size between 0.5 and 2.5 µm. This particle size increases the affinity for certain microorganisms

A preferred method for mingling the first catalyst, the second catalyst and the adsorbent is to introduce the first catalyst, the second catalyst and the adsorbent, each in a powdered state and either already pre-mingled or separate, into a liquid to form a slurry. This slurry is then preferably vigorously mixed and the liquid is evaporated to form a dry powdered mixture of the first catalyst, the second catalyst and the adsorbent. This mixture can then be used per se or to form the slurry that is coated onto the carrier.

In a detailed and preferred embodiment, in a liquid preferably consisting of 20 % alcohol and 80 % deionized water, between 5 and 10 % of titanium dioxide, between 2 and 6 % of manganese monoxide and between 10 and 20 % of the zeolite, each in regard to the total mass of the liquid, are mixed. Consecutively the mixture is heated to evaporate the liquid in order to obtain a powdered catalytic composition which can then be used. It is preferably heated to a temperature of 75 °C - 130° C for a period between 24 h and 120 h, preferably 72h.

The invention also specifically relates to a slurry, formed from the first catalyst, the second catalyst, the adsorbent and deionized water.

According to another aspect, the invention relates to a catalytic device, obtainable via a manufacturing method as described herein.

According to another aspect, the invention relates to a catalytic composition, containing, in weight percent with regard to its total mass and each in a powdered state, between 27% and 30% of a first catalyst having photocatalytic activity, between 11% and 17% of a second catalyst and between 55% and 59% of an adsorbent.

All embodiments and configurations described herein regarding the catalytic device are also preferred configurations and embodiments of the catalytic composition.

If the second catalyst, as preferred, is a low-temperature catalyst, the catalytic composition can also be called a non-thermal or a thermal catalyst, since no high heating is necessary to activate the catalyst. Since the first catalyst, the second catalyst and the adsorbent are each provided in the powdered state, the catalytic composition can also be called a powdery non-thermal or a thermal catalyst. In one embodiment, the first catalyst having photocatalytic activity is yet photo-activated.

According to a specific and preferred embodiment of the catalytic composition, the first catalyst is titanium dioxide TiO₂, the second catalyst is manganese monoxide MnO and the adsorbent is a zeolite. The zeolite preferably is a synthetic hydrophilic zeolite of type A.

Additionally, this invention relates to an embodiment of the catalytic composition, in which the amount of the first catalyst is lower than 27 % and higher than 30 %, the amount of the second catalyst is lower than 11 % and higher than 17 % and the amount of the adsorbent is lower than 55% and higher than 59%, each in weight percent and with regard to their total mass. All further preferred configurations and embodiments described herein apply to this specific embodiment.

### Reference list:

- 1: air handling unit
- 2: air guiding tubing
- 3: fan
- 4: flow direction
- 5: pre-filter
- 6: insert device
- 7: filter unit
- 8: frame casing
- 9: open end faces
- 10: UV lamps
- 11: seats
- 12: (catalytic) grid structures
- 13: brackets
- 14: screw
- 15: (standard) support frame
- 16: brackets
- 17: carrier grid
- 18: further layer
- 19: wire grid
- 20: holding frame

## Claims

1. Insert device for an air conditioning installation which air conditioning installation comprises an air handling unit (1) guiding air flowing in a given flow direction where the air handling unit (1) has a casing with a predetermined cross section in which at least a filter (5, 7) is housed, **characterized in that** the insert device comprises a frame casing (8) with an outer closed periphery shaped to be mounted within a predetermined cross section of the casing of the air handling unit (1) and has end faces (9) open for the air flow, at each of the end faces (9) an air-permeable catalytic grid structure (12) is held comprising a carrier grid (17) and a coating with a catalytic material,
that the catalytic material is a mixture comprising an absorbent, a first catalyst activatable by electromagnetic radiation, and a second catalyst being activated at low temperature,
and that the frame casing (8) in addition holds an electromagnetic radiation source device between the catalytic grid structures (12) at its end faces (9).

2. Insert device according to claim 1, **characterized in that** the first catalyst is activatable by UV radiation and that the electromagnetic radiation source emits UV radiation.

3. Insert device according to claim 2, **characterized in that** at least one UV lamp (10) is arranged between the catalytic grid structures (12) as said electromagnetic radiation source device.

4. Insert device according to claim 2, **characterized in that** a plasma generator which generates UV radiation besides a plasma serves as the electromagnetic radiation source device.

5. Insert device according to one of the claims 1 to 4, **characterized in that** both catalysts as well as the absorbent are mixed in a pulverulent condition each before coating.

6. Insert device according to one of the claims 1 to 5, **characterized in that** it comprises an air filter material.

7. Insert device according to one of the claims 1 to 6, **characterized in that** it comprises a UV blocking layer.

8. Air conditioning installation comprising in air handling unit (1) guiding air flowing in a given flow direction wherein the air handling unit (1) has a casing with a predetermined cross section in which at least a filter (5, 7) is housed, the filter (5, 7) being held by a support frame (15) having a peripheral shape adapted to the free cross-section of the casing of the air handling unit (1), **characterized in that** the insert device (6, 6') is inserted into the support frame (15).

9. Air conditioning installation according to claim (7), **characterized in that** the insert device (6, 6') is inserted into the air handling unit (1) in addition to a filter unit (7).
